# EUROPEAN PATENT APPLICATION

(11) **EP 4 775 666 A1**
(43) Date of publication of application: **15.07.2026**
(21) Application number: 25227084.8
(22) Date of filing: 23.12.2025
(51) Int. Cl.: C12N 5/00, B01D 71/48, C12M 1/12, C12M 1/00

(54) **COMPOSITE MEMBRANE STRUCTURE**

(30) Priority: 14.01.2025 TW 114101526
(71) Applicant: Darwin Precisions Corporation, Hukou Township 303 (TW)
(72) Inventor: CHENG, Ching-Jen, 303 Hukou Township, Hsinchu County (TW); WU, Yu-Hsin, 303 Hukou Township, Hsinchu County (TW); LIN, Jui-Hua, 303 Hukou Township, Hsinchu County (TW); LIU, Pang-Chun, 303 Hukou Township, Hsinchu County (TW)
(74) Representative: Zacco Denmark A/S

(57) **Abstract**

A composite membrane structure includes a middle layer and at least one surface layer. The middle layer has a plurality of holes, and the at least one surface layer is disposed on one side of the middle layer. The material of the surface layer is different from the material of the middle layer. A plurality of holes in the middle layer pass through the middle layer and open on opposite sides of the middle layer.

## Description

### FIELD OF THE INVENTION

The present invention relates to a bionic technology, and more particularly to a membrane structure used in a bionic organ device.

### BACKGROUND OF THE INVENTION

Conventional cell culture methods have difficulty replicating the complex physiological functions of biological tissues and organs, while animal testing suffers from long experimental cycle time and high costs. Organ-on-a-chip technology reconstructs the in vivo physiological environment of organs, simulating the structures, microenvironment, and physiological functions of biological organs. Also, it enables the control with precise parameters and offers advantages, such as compact design, integration, high efficiency, and cost reduction. However, current organ-on-a-chip devices are limited by the hydrophilicity of their porous membranes, which hinders accurate replication of the in vivo microenvironment and conditions, and thus cannot achieve certain desired effects in practice.

### SUMMARY OF THE INVENTION

The present invention provides a composite membrane structure that may be used for cell or tissue attachment or culture, for simulating the in vivo microenvironment, and may be used for bionic organ devices.

In order to achieve one, some, or all of the aforementioned objectives, or other objectives, a composite membrane structure is provided according to one embodiment of the present invention. The composite membrane structure includes a middle layer and at least one surface layer. The middle layer includes a plurality of holes. The at least one surface layer is disposed on one side of the middle layer. The surface layer includes a material different from that of the middle layer. The holes pass through the middle layer and include openings in two opposite sides of the middle layer.

According to an embodiment of the present invention, the at least one surface layer includes a hydrophobic material having biocompatibility.

By using the composite structure including the intermediate layer and the surface layer made of different materials, the present invention enables the membrane to possess suitable hydrophilic and/or hydrophobic properties as well as gas permeability. When used for cell or tissue attachment or culture, it not only facilitates better simulation of the in vivo microenvironment, but also allows for various applications to meet different experimental or simulation requirements.

Other objectives, features, and advantages of the invention will be further understood from the further technological features disclosed by the embodiments of the invention wherein there are shown and described preferred embodiments of this invention, simply by way of illustration of modes best suited to carry out the invention.

### BRIEF DESCRIPTION OF THE DRAWINGS

FIG. 1 is a schematic perspective view of a composite membrane structure according to a first embodiment of the present invention;
FIG. 2 is a schematic cross-sectional view of the composite membrane structure, taken along line aa' in FIG. 1;
FIG. 3 is a schematic cross-sectional view of the composite membrane structure according to a second embodiment of the present invention; and
FIG. 4 is a schematic diagram showing an operation of the composite membrane structure according to an embodiment of the present invention.

### DETAILED DESCRIPTION OF PREFERRED EMBODIMENTS

In the following description of embodiments according to the present invention, terms indicating orientation or positional relationships, such as "upper," "lower," etc., are described based on the orientation or positional relationships shown in the corresponding drawings. These terms are used solely for the convenience of describing the invention and are not intended to limit the invention or to imply that the referenced elements must have a particular orientation or be constructed in a particular orientation. Furthermore, the terms "first," "second," and the like, as used in this specification or the claims, are employed merely to designate names of elements or to distinguish between different embodiments or ranges and are not intended to limit the number of elements. Unless limited otherwise, the terms "connected" or "coupled" as used in this specification or the claims may refer to either a direct connection between two elements without the presence of other elements or an indirect connection through the presence of other elements.

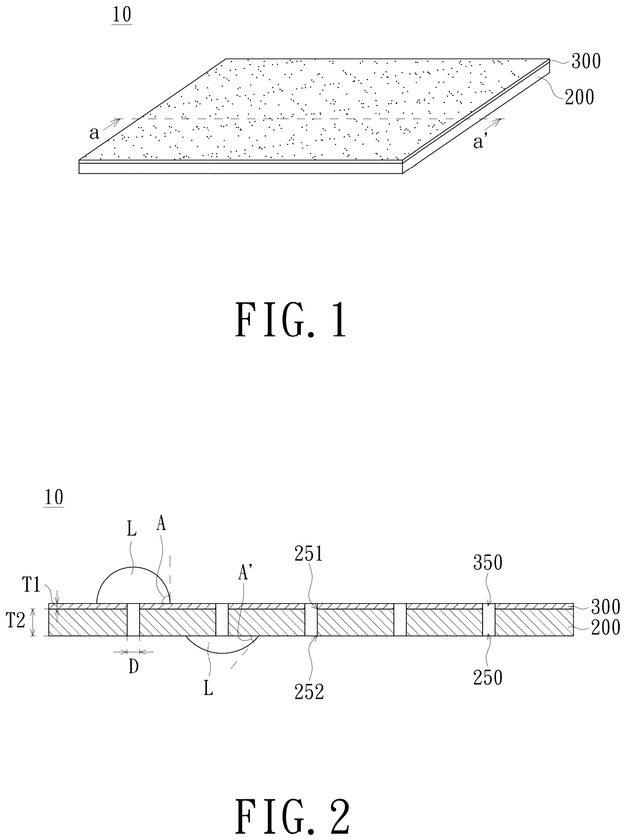

FIG. 1 is a schematic perspective view of a composite membrane structure according to a first embodiment of the present invention. FIG. 2 is a schematic cross-sectional view of the composite membrane structure, taken along line aa' in FIG. 1. As shown in FIG. 1 and FIG. 2, according to the embodiment of the present invention, a composite membrane structure 10 including a middle layer 200 and at least one surface layer 300 is provided. The middle layer 200 includes a plurality of holes 250. The surface layer 300 is disposed on one side of the middle layer 200. The surface layer 300 includes a material different from a material of the middle layer 200. In the present embodiment, a thickness T1 of the surface layer 300 is smaller than a thickness T2 of the middle layer 200. For example, the surface layer 300 may have the thickness T1 in a scale of nanometers (nm), such as dozens or hundreds of nanometers. The middle layer 200 may have the thickness T2 in a scale of micrometers (µm), such as several micrometers, tens of micrometers, or dozens of micrometers. In the embodiment shown in FIG. 1 and FIG. 2, a quantity of the surface layer 300 is one. However, the quantity of the surface layer 300 may be two in other embodiments (described in the following). The two surface layers 300 may be disposed on two opposite sides of the middle layer 200. In other words, a sandwich structure might be formed by the two surface layers 300 and the middle layer 200 therebetween.

As shown in FIG. 2, the holes extend through the middle layer 200 and form openings 251 and 252 on the two opposite sides of the middle layer 200. The diameters of the openings 251 and 252 may be the same or different. The holes 250 may have a diameter D in the scale of nanometers, such as dozens or hundreds of nanometers. In an embodiment of the invention, the diameter D of the holes 250 may be, for example, between 200 nm and 800 nm, such as 200 nm, 300 nm, 400 nm, 500 nm, 600 nm, 700 nm, or 800 nm, but the invention is not limited thereto. As shown in FIG. 1, the holes 250 may be distributed uniformly in the middle layer 200. A distribution density may be, for example, tens of thousands, hundreds of thousands, or millions holes per square centimeter. In an embodiment of the present invention, the distribution density of the holes 250 is, for example, between 5×10⁵ and 8×10⁶ holes per square centimeter, such as 5×10⁵, 1×10⁶, 2×10⁶, 3×10⁶, 4×10⁶, 5×10⁶, 6×10⁶, 7×10⁶, or 8×10⁶ holes per square centimeter. The invention is not limited thereto. The distribution density of the holes 250 and the size of the holes 250 may be designed correspondingly to provide a porosity within an appropriate range, such that the middle layer 200 and the composite membrane structure 10 may have suitable gas permeabilities.

In the embodiment of the present invention, the middle layer 200 and the surface layer 300, for example, are made of materials having biocompatibility. The materials may be polymers. For example, the middle layer 200 may be made of polyethylene terephthalate (PET), polydimethylsiloxane (PDMS), polyurethane, styrene-ethylene-butylene-styrene (SEBS), poly(2-hydroxyethyl methacrylate) (pHEMA), polyethylene glycol (PEG) or polyvinyl alcohol (PVA), or polycarbonate (PC). The invention is not limited thereto. In the embodiment of the present invention, the thickness T2 may be 10 µm, 15 µm, 20 µm, 25 µm, 30 µm, 35 µm, 40 µm, 45 µm, or 50 µm, but is not limited to. The surface layer 300 generally has the thickness T1 thinner than the thickness T2 and also has a greater hydrophobicity in comparison with the middle layer 200. For example, the middle layer 200 may be hydrophilic while the surface layer 300 is hydrophobic; or when the surface layer 300 and the middle layer 200 are both hydrophobic, the surface layer 300 has greater hydrophobicity than the middle layer. As shown in FIG. 2, liquid L has a contact angle A with the surface layer 300 (as shown in the upper part of FIG. 2) greater than a contact angle A' with the middle layer 200 (as shown in the lower part of FIG. 2). In an embodiment of the present invention, the contact angle A formed between the surface layer 300 and the liquid L is greater than 90 degrees while the contact angle A' between the middle layer 200 and the liquid L may be, for example, between 20 and 40 degrees. The invention is not limited thereto. Accordingly, the composite membrane structure 10 according to the embodiment of the present invention may have different hydrophobicity on the two sides of the composite membrane structure 10.

The surface layer 300 may be made of a material selected from polymers, such as polyethylene terephthalate (PET), polydimethylsiloxane (PDMS), polyurethane, styrene-ethylene-butylene-styrene (SEBS), poly(2-hydroxyethyl methacrylate) (pHEMA), polyethylene glycol (PEG) or polyvinyl alcohol (PVA), polycarbonate (PC), or determined according to the material of the middle layer 200. The invention is not limited thereto. In some embodiments, when the middle layer 200 is made of PET, the surface layer 300 may be made of poly-p-xylylene or the like. The surface layer 300 may be directly formed on one side or two opposite sides of the middle layer 200, or the surface layer 300 may be first formed separately and then attached onto the middle layer 200. In one embodiment, the surface layer 300 is formed directly on the middle layer 200 through a process, such as coating, deposition, or printing. In one embodiment of the present invention, a deposition process is utilized to deposit the material molecules on the middle layer 200 to form the surface layer 300.

As shown in FIG. 1 and FIG. 2, the surface layer 300 further includes a plurality of pores 350. The pores 350 correspond to the holes 250 in the middle layer 200, respectively. In the embodiment of the present invention, the pores 350, for example, are simultaneously formed with the formation of the surface layer 300. In addition, in an embodiment of the present invention, the locations of the pores 350 may be determined based on the locations of the holes 250 in the middle layer 200. For example, when the material of the surface layer 300 is deposited on the middle layer 200 during the deposition process, gaps may be left at the locations above the holes 250 since the material molecules could not be deposited on the holes 250, thereby forming the pores 350 in the surface layer 300.

In the embodiment of the present invention, the thickness T1 of the surface layer 300 may be further designed based on the dimensions of the holes 250. For example, the thickness T1 of the surface layer 300 may be designed as a certain percentage of the diameter D of the holes 250, such as less than 100% of the diameter D. In an embodiment, the thickness T1 is, for example but not limited to, between 20% and 60% of the diameter D. For example, in case the diameter D of the holes 250 is 200 nm, the thickness T1 of the surface layer 300 is, for example, between 40 nm and 120 nm. In case the diameter D of the holes 250 is 500 nm, the thickness T1 of the surface layer 300 is, for example, between 100 nm and 300 nm. In case the diameter D of the holes 250 is 800 nm, the thickness T1 of the surface layer 300 is, for example, between 160 nm and 480 nm. In addition, the lower and upper limits of the percentage may vary depending on the materials of the surface layer 300 and the middle layer 200.

In the embodiment of the present invention, the surface layer 300 is made of a specific material, such as poly-p-xylylene or the like, and the thickness T1 is based on a lower limit, for example, at least 20% of the diameter D of the holes 250, thereby ensuring the formed contact angle A greater than 90 degrees. As the thickness T1 of the surface layer 300 increases, the contact angle A can be effectively increased to, for example, 95 degrees, 100 degrees, 105 degrees, 110 degrees, or more. However, the thickness T1 of the surface layer 300 is less than an upper limit, such as 60% of the diameter D of the holes 250. In some embodiments, it helps to ensure that the gaps above the holes 250 are sufficient to form pores 350 with proper dimensions, such that the composite membrane structure 10 may have a porosity within a proper range accordingly. The invention is not limited thereto. For example, even if the integrity of the holes 250 may be affected during the material deposition process, such as the material deposited within or around the holes 250, the invention ensures the hydrophobicity and the porosity of the composite membrane structure 10 by defining the lower and upper limits of the thickness T1 of the surface layer 300 and by controlling the deposition in a proper extent. The hydrophobicity of the surface layer 300 of the composite membrane structure 10 causes liquid to form droplets, making it less likely to pass through the composite membrane structure 10, while the holes 250 allow gas to pass through. Accordingly, the composite membrane structure 10 according to the embodiment of the present invention provides both hydrophobicity and gas permeability.

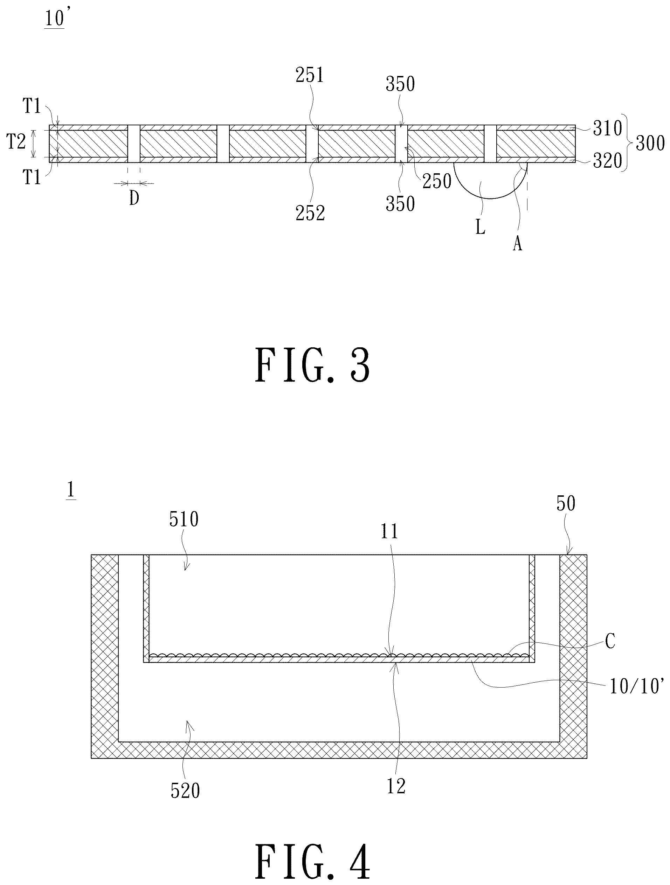

FIG. 3 is a schematic cross-sectional view of the composite membrane structure according to another embodiment of the present invention. As shown in FIG. 3, the surface layer 300 of the composite membrane structure 10' may further include a first surface layer 310 and a second surface layer 320. The first surface layer 310 and the second surface layer 320 are disposed on the two opposite sides of the middle layer 200. The thickness T1 of the first surface layer 310, the thickness T1 of the second surface layer 320, and the thickness T2 of the middle layer 200 are as described previously and will not be reiterated here. The thickness T1 of the first surface layer 310 and the thickness T1 of the second surface layer 320 may be designed as described previously, i.e., based on the dimensions of the holes 250, and are a certain percentage of the diameter D of the holes 250, respectively. The first surface layer 310 and the second surface layer 320 are made of materials as mentioned above. In addition, in one embodiment, the material of the first surface layer 310 is the same as the material of the second surface layer 320. The invention is not limited thereto. Furthermore, the quantity of the surface layer 300 is not limited to one or two. For example, a plurality of surface layers 300 may be stacked on one side of the middle layer 200. The materials of these surface layers 300 may be the same or different.

The first surface layer 310 and the second surface layer 320 have greater hydrophobicity in comparison with the middle layer 200. As aforementioned, the first surface layer 310 and the second surface layer 320 may form contact angles greater than 90 degrees with the liquid L. In addition, when the first surface layer 310 and the second surface layer 320 are made of different materials, the first surface layer 310 and the second surface layer 320 may have different hydrophobicity. However, the invention is not limited thereto. Accordingly, the composite membrane structure 10' of the present embodiment may have different hydrophobicity on the two sides. The first surface layer 310 and the second surface layer 320 may include a plurality of pores 350. The pores 350 correspond to the holes 250 respectively. For example, the openings 251 and 252 in any of the holes 250 respectively correspond to the pores 350 in the first surface layer 310 and the pores 350 in the second surface layer 320. In an embodiment of the present invention, the pores 350 in the first surface layer 310 and/or the second surface layer 320 are, for example, formed simultaneously during the formation of the first surface layer 310 and/or the second surface layer 320. Furthermore, the first surface layer 310 and the second surface layer 320 may be formed in sequence. For example, varied materials may be deposited on the two sides of the middle layer 200 sequentially to form the first surface layer 310 and the second surface layer 320.

The composite membrane structures 10 and 10' according to the embodiments of the present invention may be used for cell or tissue attachment or culture, and/or used in bionic organ devices. The hydrophobicity of the composite membrane structure 10/10' helps to create an interface layer, such as a gas-liquid interface layer, which allows gas to pass through but blocks passage of liquid. In addition, the composite membrane structure 10/10' having different hydrophobicity on the two sides can further make the interface layer directional, for example, impeding liquid passage in a specific direction.

FIG. 4 is a schematic diagram showing an operation of using the composite membrane structure 10/10' in a bionic organ device according to an embodiment of the present invention. As shown in FIG. 4, a bionic organ device 1 may be provided with a case 50 and the composite membrane structure 10/10'. In the case 50, a plurality of spaces may be defined, such as a first chamber 510 and a second chamber 520. The first chamber 510 and the second chamber 520 are separated by the composite membrane structure 10/10'. One side of the composite membrane structure 10' (first surface 11), facing the first chamber 510, is adapted to attach or culture cells C, and the other side of the composite membrane structure 10' (second surface 12), facing the second chamber 520, is adapted to attach or culture another kind of cells (not shown). In addition, different liquids (not shown), such as culture media with different compositions, may be disposed in the first chamber 510 and the second chamber 520. Alternatively, liquid and gas may be placed in the first chamber 510 and the second chamber 520, respectively. For example, the liquid is placed in the first chamber 510 and the gas is placed in the second chamber 520; or the gas is placed in the first chamber 510 and the liquid is placed in the second chamber 520. Accordingly, the bionic organ device 1 can simulate the in vivo microenvironment and further test or simulate the interactions between different kinds of cells.

In some embodiments, one side of the composite membrane structure 10/10', such as the first surface 11, may be used to attach or culture, for example, pulmonary epithelial cells, and the first chamber 510 may be configured to simulate the in vivo environment of pulmonary epithelial cells, including the delivery of specific gases, such as oxygen-containing gases. The second surface 12 may be used to attach other kinds of cells, such as vascular endothelial cells. The invention is not limited thereto. In this case, the second chamber 520 is configured to simulate the in vivo environment of vascular endothelial cells, including the delivery of culture media. According to the gas permeability of the composite membrane structure 10/10', gas can pass through the composite membrane structure 10/10' between the first chamber 510 and the second chamber 520 while the hydrophobicity of the composite membrane structure 10/10' may restrict or avoid the passage of liquid.

As described above, the composite membrane structure 10/10' of the present invention is adapted to provide gas exchange to the attached or cultured cells between both sides of the membrane structure due to its gas permeability. On the other hand, the composite membrane structure 10/10' may function as a selective membrane. As a selective membrane, the composite membrane structure 10/10' may simulate a function of a natural barrier, such as no communication between liquids in tissues of a body, or can be applied in bionic organ devices according to experimental design.

While the invention has been described in terms of what is presently considered to be the most practical and preferred embodiments, it is to be understood that the invention needs not be limited to the disclosed embodiment. On the contrary, it is intended to cover various modifications and similar arrangements included within the spirit and scope of the appended claims which are to be accorded with the broadest interpretation so as to encompass all such modifications and similar structures.

## Claims

1. A composite membrane structure (10), comprising:
a middle layer (200), comprising a plurality of holes (250); and
at least one surface layer (300), disposed on one side of the middle layer (200), wherein the at least one surface layer (300) comprises a material different from a material of the middle layer (200);
wherein the holes (250) in the middle layer (200) extend through the middle layer (200) and have openings (251, 252) on two opposite sides of the middle layer (200).

2. The composite membrane structure (10') according to claim 1, **characterized in that** the at least one surface layer (300) further comprises a first surface layer (310) and a second surface layer (320), and the first surface layer (310) and the second surface layer (320) are composed of hydrophobic materials and disposed on the two opposite sides of the middle layer (200), respectively.

3. The composite membrane structure (10) according to claim 1, **characterized in that** the holes (250) are distributed uniformly in the middle layer (200) and have a distribution density between 5×10⁵ and 8×10⁶ holes per square centimeter.

4. The composite membrane structure (10) according to claim 1, **characterized in that** the holes (250) have a diameter between 200 nm and 800 nm.

5. The composite membrane structure (10) according to claim 4, **characterized in that** the at least one surface layer (300) has a thickness less than the diameter of the holes (250).

6. The composite membrane structure (10) according to claim 5, **characterized in that** the thickness of the at least one surface layer (300) is from 20% to 60% of the diameter of the holes (250).

7. The composite membrane structure (10) according to claim 1, **characterized in that** the at least one surface layer (300) comprises a hydrophobic material having biocompatibility.

8. The composite membrane structure (10) according to claim 7, **characterized in that** the at least one surface layer (300) is adapted to allow liquid (L) to form a contact angle (A) greater than 90 degrees.

9. The composite membrane structure (10) according to claim 7, **characterized in that** a hydrophobicity of the at least one surface layer (300) is greater than a hydrophobicity of the middle layer (200).

10. The composite membrane structure (10) according to claim 1, **characterized in that** the at least one surface layer (300) comprises a plurality of pores (350) corresponding to the holes (250) respectively, and sizes of the pores (350) are less than or equal to sizes of the holes (250).
